Europäisches Patentamt
European Patent Office
Office européen des brevets

(19)

(11) Numéro de publication: **0 042 348**
**A1**

## (12) DEMANDE DE BREVET EUROPEEN

(21) Numéro de dépôt: **81400972.6**

(22) Date de dépôt: **17.06.81**

(51) Int. Cl.³: **A 61 K 31/475**, C 07 D 471/04
// (C07D471/04, 221/00, 209/00)

(30) Priorité: **18.06.80 FR 8013565**

(43) Date de publication de la demande: **23.12.81**
**Bulletin 81/51**

(84) Etats contractants désignés: **AT BE CH DE GB IT LI LU NL SE**

(71) Demandeur: **ANVAR Agence Nationale de Valorisation de la Recherche, 43, rue Caumartin, F-75436 Paris Cedex 09 (FR)**

(72) Inventeur: **Husson, Henri-Philippe, 20, route de Milan, F-78460 Chevreuse (FR)**
Inventeur: **Bessellevre, Richard, 160, Avenue du Général Leclerc- Bât.10, F-91190 Gif Sur Yvette (FR)**
Inventeur: **Potier, Pierre, 5, rue Lafontaine, F-78390 Bois D'Arcy (FR)**
Inventeur: **Le Pecq, Jean-Bernard, 37, Square Saint-Charles, F-75012 Paris (FR)**
Inventeur: **Paoletti, Claude, 84, rue Michel Ange, F-75016 Paris (FR)**

(74) Mandataire: **Phélip, Bruno et al, c/o Cabinet HARLé & LECHOPIEZ 21, rue de La Rochefoucauld, F-75009 Paris (FR)**

# (54) Dérivés de l'olivacine et leur application thérapeutique.

(57) Nouveaux dérivés d'olivacine.
Ils répondent à la formule:

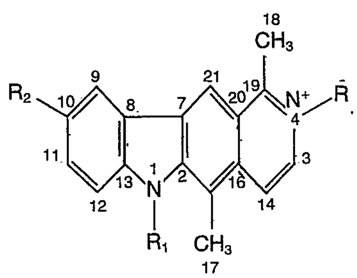

$X^-$ (I)

dans laquelle:

R est un atome d'hydrogène, ou un groupe alkyle, alkylamino ou aralkyle, substitué ou non, ou rien et dans ce dernier cas l'atome d'azote en position 4 n'est pas quaternarisé;

$R_1$ représente un atome d'hydrogène ou un groupe alkyle, alkylamino, ou aralkyle, substitué ou non;

$R_2$ représente un atome d'hydrogène ou un groupe alcoxy ou hydroxy;

à l'exception des cas où on a simultanément $R_1 = R_2 = H$ quelque soit R d'une part, et R = rien, $R_1 = H$, $R_2 = OH$ ou $OCH_3$ d'autre part; et $X^-$ représente un anion d'acide minéral ou organique, ou rien et le composé correspondant est alors une base libre, ainsi que les sels d'addition des bases libres de formule 1 avec des acides pharmaceutiquement acceptables.

Application au traitement des cancers.

EP 0 042 348 A1

0042348

Dérivés de l'olivacine et leur application thérapeutique.

La présente invention concerne, à titre de nouveaux produits des dérivés de l'olivacine et l'application de ces composés comme médicament ou en tant que principe actif de compositions pharmaceutiques, en particulier pour le traitement des cancers.

Les composés selon l'invention répondent à la formule développée plane :

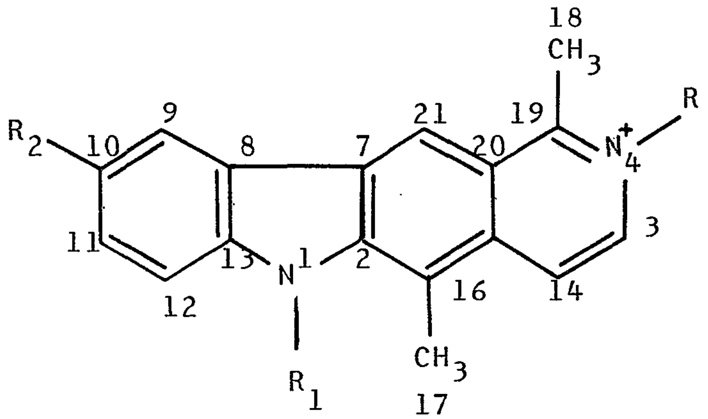

$X^-$ (I)

dans laquelle : R est un atome d'hydrogène, ou un groupe alkyle, alkylamino, ou aralkyle, substitué ou non, ou rien, et dans ce dernier cas l'atome d'azote en position 4 n'est pas quaternarisé ;

$R_1$ représente un atome d'hydrogène ou un groupe alkyle, alkylamino, ou aralkyle, substitué ou non ;

$R_2$ représente un atome d'hydrogène, ou un groupe alcoxy ou hydroxy ;

à l'exception des cas où on a simultanément $R_1 = R_2 = H$ quel que soit R d'une part et R = rien, $R_1 = H$, $R_2 = OH$ ou $OCH_3$ d'autre part ; et

$X^-$ représente un anion d'acide minéral ou organique, ou rien et le composé correspondant est alors une base libre,

et comprennent également les sels d'addition avec un acide pharmaceutiquement acceptable des

bases libres correspondantes.

La numérotation des atomes constituant le squelette de base de ces composés est celle des alcaloïdes indoliques préconisée par J. Le Men et W.I. Taylor, Experientia 21, 508 (1965) et établie pour l'olivacine par P. Potier et M.M. Janot, C.R. Acad. Sci, 276 C, 1727 (1973).

On a préparé les composés selon l'invention à partir de l'un des deux alcaloïdes que sont respectivement l'olivacine (R = rien et $R_1 = R_2 = H$ dans la formule ci-dessus) et la méthoxyolivacine (R = rien, $R_1 = H$ et $R_2 = OCH_3$ dans la formule ci-dessus).

L'olivacine a été isolée et caractérisée par J. Schmutz et F. Hunziecker dans Pharm. acta Helv. 33, 341 (1958). La méthoxyolivacine a été isolée et décrite pour la première fois par R.H. Burnell et D. Della Casa dans Can. J. Chem. 45, 89 (1967).

De nos jours on sait obtenir ces deux alcaloïdes soit par synthèse ou par hémisynthèse, notamment par les méthodes décrites par R. Besselièvre dans sa Thèse de Doctorat d'Université, Université de Paris Sud, Centre d'Orsay, 24 mai 1977, et par R. Besselièvre et H.-P. Husson dans Tetrahedron Letters, 1873 (1976).

On peut préparer les composés de formule I selon l'invention en faisant réagir avec, soit l'olivacine soit la méthoxyolivacine susdites, un ou plusieurs des réactifs connus de l'homme de l'art comme appropriés pour permettre l'introduction sur l'une ou plusieurs des positions 1 et/ou 4 de la structure d'olivacine du (ou des) groupe(s) approprié(s) pour l'obtention du composé de formule I recherché, ou d'un précurseur de celui-ci s'il n'est pas obtenu directement à partir de l'olivacine ou de la méthoxyolivacine. Par exemple, on peut remplacer par des groupes alkyle les atomes d'hydrogène des positions 1 et/ou 4 par réaction de l'olivacine ou de la méthoxyolivacine avec un agent d'alkylation tel qu'un halogénure d'alkyle, en présence d'une base ou non. Pour fixer des groupes aralkyle sur au moins l'une de ces positions, il convient de recourir à un halogénure d'aralkyle approprié et d'opérer également, de façon connue, en présence d'une base. Pour introduire des groupes alkylamino, on utilise classiquement des halogénures d'aminoalkyle, substitués ou non, comme par exemple du β -chloro diéthylaminoéthyle, dans le diméthylformamide.

L'hydroxylation ou l'alcoxylation sur la position 10, quand elle est souhaitée, peut être réalisée par l'une quelconque des techniques connues pour respectivement l'hydroxylation ou l'alcoxylation sur des systèmes à cycles aromatiques condensés. De préférence cependant, les composés portant un groupe hydroxy en 10 peuvent être obtenus par déméthylation de la méthoxyolivacine en présence de chlorhydrate de pyridinium.

En ce qui concerne la quaternarisation éventuelle de l'atome d'azote en position 4, elle est réalisée dans la pratique au moyen de tout agent alkylant connu comme apte à quaternariser un atome d'azote tertiaire (par exemple un halogénure, tel qu'un bromure,un iodure ou un chlorure, un tosylate, un mésylate ou un sulfate approprié, entre autres). Au moyen d'un acide organique ou minéral, on peut obtenir un sel d'addition avec le composé de formule I non quaternarisé. Dans le cas où le composé quaternarisé en 4 qui en résulte n'est pas lui-même soluble dans l'eau, il convient en pratique de le soumettre en outre à un échange d'ions pour le rendre hydrosoluble, en particulier en vue d'en

faciliter l'application en thérapeutique humaine ou animale.

Pour plus de précisions, il convient de se reporter aux exemples illustratifs détaillés plus loin et qui enseignent concrètement comment on peut préparer certains des composés de formule I qui sont en fait les composés préférés conformes à l'invention.

Pour l'isolement du composé recherché, on peut utiliser toute technique de séparation appropriée comme la filtration, la décantation, etc... et on peut, si on le désire, purifier les produits ainsi isolés, par exemple par cristallisation ou recristallisation.

Dans le présent texte, on entend par groupe "alkyle", tout groupe hydrocarboné saturé, linéaire ou ramifié, contenant de préférence de 1 à 5 atomes de carbone. On entend par groupe "aralkyle" tout groupe hydrocarboné insaturé composé essentiellement d'un système aromatique à un ou plusieurs cycles benzéniques et portant au moins un groupe alkyle tel que susdit. On entend par groupe "alkylamino" tout groupe alkyle tel que susdit substitué par un groupe amino lui-même éventuellement alkylé. Enfin, on entend par groupe "alcoxy" tout groupe éther d'alkyle dans lequel le groupe alkyle est tel que décrit ci-dessus.

Les composés selon l'invention sont utiles comme principe actif de médicaments antitumoraux et se sont révélés actifs contre de nombreuses tumeurs, en particulier contre la leucémie murine L 1210.

Parmi ces composés, on préfère plus particulièrement les acétates et les lactates de :

4 - méthylolivacinium,

1,4 - diméthyl olivacinium,

10 - hydroxy olivacinium,

4 - méthyl 10 - hydroxy olivacinium,
4 - méthyl 10 - méthoxy olivacinium,
4 - ( β -diéthylaminoéthyl-) 10 - hydroxy olivacinium,

ainsi que la 10-hydroxy olivacine base, ou tout sel d'addition avec un acide pharmaceutiquement acceptable des bases correspondantes.

La préparation de ces composés ou de leurs précurseurs est décrite plus concrètement dans les exemples ci-après, qui ne limitent aucunement l'invention.

EXEMPLE 1. Acétate de 4-méthyl olivacinium.

On a dissous 4,750 g d'olivacine dans 600cm³ de méthanol au reflux, on a ajouté 20 cm³ d'iodure de méthyle et on a maintenu au reflux pendant 40 heures. On a refroidi et on laissé reposer une nuit ; on a ensuite essoré, lavé et séché, obtenant ainsi 4,6 g de cristaux verts d'iodométhylate d'olivacine. On a remis ces cristaux en suspension dans 400 cm³ d'eau et on a ajouté 60 cm³ de résine essorée d'échangeur d'ions de type Amberlite IRA 400 sous forme acétate. On a agité pendant 18 heures, après quoi l'iodométhylate était entièrement solubilisé. On a filtré la suspension de résine au travers d'une colonne contenant 30 cm³ de résine Amberlite IRA 400 (commercialisée sous cette dénomination par Rohm et Haas) sous forme acétate,et on a ensuite rincé à l'eau.

Le filtrat a été amené à sec sous vide et repris par 150 cm³ de méthanol. On a filtré la solution verte obtenue sur une colonne contenant 300 cm³ de poudre de cellulose Whatman, retenant ainsi un pigment bleu. On a concentré jusqu'à faible volume le filtrat jaune obtenu et on a amorcé une belle cristallisation par addition

d'éther. On a laissé reposer environ 48 heures, puis on a essoré, lavé à l'éther et séché, obtenant ainsi 1,7 g de cristaux jaune vif d'acétate de 4-méthyl olivacinium, qu'on a recristallisés dans un mélange méthanol-éther pour analyse.

Point de fusion F (au bloc Kofler) : décomposition à 228°C.

Analyse : (dans les calculs, on a considéré qu'il y avait une molécule de méthanol de solvatation).

| $C_{21}H_{24}N_2O_3$ | C | H | N | O |
|---|---|---|---|---|
| Calculé | 71,59 | 6,82 | 7,95 | 13,64 |
| Trouvé | 70,60 | 7,01 | 7,82 | 12,81 |

EXEMPLE 2. 1-méthyl olivacine

On a porté 50mg d'olivacine et 100 mg d'hydroxyde de tétraméthylammonium lyophilisé à 220°C au bain métallique, sous une pression réduite de 0,1 mm de mercure, dans un appareil à sublimer, pendant 3 heures. On a isolé 38 mg de sublimé brun, semi-cristallisé, qu'on a recristallisé dans le méthanol. On a ainsi isolé 20 mg de cristaux jaunes que l'on a séchés sous vide.

F = 224°C

le spectre de masse a donné un $M^+$ à m/e 260.

Spectre de RMN $^1H$ ($CDCl_3$ - $CD_3OD$)

$\delta$ TMS = 0

s 2,83 (3H17), s 2,91 (3H18),

s 3,93 ($CH_3$1), m 7,25 - 8,30 6H aromatique,

s 8,50 (1H21).

EXEMPLE 3. Iodure de 1,4-diméthylolivacinium.

On a porté 6,6 mg de 1-méthyl olivacine, obtenu comme décrit dans l'exemple 2, au reflux dans 3 $cm^3$ d'acétonitrile, en présence de 0,3 $cm^3$ d'iodure de méthyle. Au bout d'une heure de reflux, on a constaté que le produit de départ était entièrement

transformé (comme l'a montré un contrôle par chromatographie en couche mince sur silice, avec pour solvant du chlorure de méthylène à 10 % de méthanol).

On a ensuite amené à sec sous vide, ce qui a fait apparaître des cristaux jaunes, qu'on a recristallisés dans l'éther. L'isolement de ces cristaux a fourni 6 mg d'iodure de 1,4-diméthyl olivacinium.

F $>$ 260°C

On obtenait une réaction des halogènes positive au test de Beilstein.

Analyse : $C_{19}H_{19}N_2I$

| | C | H | N | I |
|---|---|---|---|---|
| Calculé | 56,72 | 4,73 | 6,97 | 31,59 |
| Trouvé | 57,01 | 4,95 | 6,55 | -- |

EXEMPLE 4. 10-hydroxy olivacine

On a porté au reflux pendant 1 heure 20 minutes dans un bain métallique 2,7 g de méthoxy olivacine dans 27 g de chlorhydrate de pyridinium fondu. On a laissé refroidir et on a ajouté 200 $cm^3$ d'eau saturée de bicarbonate de sodium. On a agité durant une nuit. On a essoré le précipité formé et on a lavé abondamment avec de l'eau à 70°C. On a isolé, après séchage à poids constant, 2,210 g de précipité brun de 10-hydroxy olivacine, dont on a sublimé un échantillon pour analyse (à 200°C sous 0,1 mm de mercure).

F de l'acétate : décomposition à 213°C

F de la base : $>$ 260°C

Spectre UV :

nm ($\varepsilon$) EtOH : 229 (24 844), 248 (25 974), 277(37 267), 301 (50 818), 338 (12 422)

EtOH + NaOH : 254 (35 000), 274 (30 491), 313 (42 913), 345 (23 715).

Spectre de masse : $M^+$ à m/e 262.

EXEMPLE 5. Acétate de 4-méthyl 10-hydroxy olivacinium.

On a mis en suspension dans 300 cm³ de méthanol 5 g de 10-hydroxy olivacine, on a ajouté 5 cm³ d'iodure de méthyle et on a porté au reflux sous agitation pendant une nuit. On a refroidi et essoré les cristaux jaune vif d'iodométhylate de 10-hydroxy olivacine ; on a isolé après séchage 4,340g de ces cristaux, qu'on a remis en suspension dans 400cm³ d'eau. On a ajouté 30 cm³ de résine Amberlite lRA 400 sous forme acétate. On a agité durant deux heures,au bout desquelles on a observé la dissolution du sel. On a ensuite filtré sur une colonne contenant 20 cm³ de résine Amberlite IRA 400 sous forme acétate. On a rincé et amené le filtrat à sec sous vide.On a redissous l'extrait sec dans 30 cm³ de méthanol et on a ajouté de l'éther pour faire apparaître une belle cristallisation orange. On a laissé reposer une nuit et ensuite on a essoré, lavé à l'éther et séché les cristaux (qui sont particulièrement hygroscopiques). On a obtenu 4,634 g de produit en cristaux ayant un point de fusion F de 250°C (décomposition).

Analyse : $C_{21}H_{24}N_2O_4$ (produit solvaté par une molécule de méthanol)

| | C | H | N | O |
|---|---|---|---|---|
| Calculé | 68,48 | 6,52 | 7,61 | 17,39 |
| Trouvé | 69,01 | 5,90 | 7,35 | 16,88 |

EXEMPLE 6. Acétate de 4-méthyl 10-méthoxy olivacinium

On a dissous au reflux dans 600 cm³ de méthanol 5 g de méthoxy olivacine. On a ajouté 20 cm³ d'iodure de méthyle et on a maintenu au reflux pendant 24 heures. On a refroidi, essoré et lavé les cristaux d'iodométhylate de 10-méthoxy olivacine obtenus. On a remis ces cristaux en suspension dans 400 cm³ d'eau, on a ajouté 30 cm³ de résine Amberlite

IRA 400 sous forme acétate. On a agité pendant 2 heures, au bout desquelles le sel était dissous. On a ensuite filtré à travers une colonne contenant 20 $cm^3$ de résine Amberlite/IRA 400 sous forme acétate, et on a amené le filtrat à sec. On a repris l'extrait sec par 10 $cm^3$ de méthanol et on a ajouté de l'éther jusqu'à l'apparition d'une cristallisation. On a laissé reposer une nuit ; on a essoré, lavé avec de l'éther et séché sous vide. On a obtenu 6,220 g de cristaux jaune-orange d'acétate de 4-méthyl 10-méthoxy olivacinium, ayant un point de fusion F de 180°C (décomposition).

Analyse : $C_{22}H_{26}N_2O_4$ (solvaté par une molécule de méthanol)

| | C | H | N | O |
|---|---|---|---|---|
| calculé | 69,11 | 6,81 | 7,33 | 16,75 |
| trouvé | 68,87 | 6,93 | 6,95 | 17,04 |

EXEMPLE 7 . Chlorure de 4-($\beta$ -diéthylaminoéthyl) 10-hydroxy olivacinium.

On a dissous 0,5 g de 10-hydroxy olivacine dans 8 $cm^3$ de diméthylformamide redistillé. On a ajouté 1 g de $\beta$ -chloro diéthylaminoéthyle base et on a laissé sous agitation pendant une semaine. On a ajouté de l'éther et on a essoré le précipité obtenu, qu'on a ensuite lavé abondamment à l'éther. On a séché sous vide et obtenu 0,43 g de poudre ocre, qu'on a recristallisée dans le mélange méthanol-éther.

On a isolé 0,310 g de chlorure de 4-($\beta$ -diéthylaminoéthyl) 10-hydroxy olivacinium, ayant un point de fusion F $>$ 260°C.

Analyse : $C_{24}H_{32}N_3O_2Cl$ (avec une molécule de méthanol de solvatation).

| | C | H | N | O | Cl |
|---|---|---|---|---|---|
| Calculé | 67,05 | 7,45 | 9,78 | 7,45 | 8,27 |
| Trouvé | 66,71 | 7,01 | 10,03 | 7,01 | 8,94 |

L'activité antitumorale des composés selon l'invention a pu être démontrée, notamment sur des leucémies murines L 1210 ; on a ainsi pu établir que les composés nouveaux selon l'invention, et en particulier les composés préférés susdits, présentent une efficacité tout à fait exceptionnelle pour les traitements anticancéreux des êtres vivants.

La tumeur expérimentale qu'est la leucémie L 1210 de la souris est en effet couramment utilisée pour l'évaluation de tous les composés antitumoraux actuellement mis en oeuvre en thérapeutique humaine, ainsi qu'il est décrit, par exemple, par C.C. ZUBROD dans Proc. Nat. Acad. Sci. USA 69, 1972, pp. 1042-1047. Le système tumoral ainsi constitué expérimentalement permet une évaluation quantitative très précise de l'activité du composé testé et par conséquent aussi une comparaison objective entre les activités respectives de différents composés, par exemple selon les méthodes décrites par H.E. SKIPPER, F.M. SCHABEL Jr. et W.S. WILCOX dans Cancer Chemother. Rep., 35, 1964, pp.1-111 et 45, 1965, pp. 5-28.

En pratique, on a utilisé des souris ayant reçu par voie intrapéritonéale un inoculum de cellules de leucémie L 1210 et ayant été, pour la moitié d'entre elles, soumises 24 heures après ladite inoculation tumorale à une injection intrapéritonéale d'une dose unique de l'un des composés à tester ; l'autre moitié des souris a reçu une injection d'un volume identique d'un solvant inactif et servait de série témoin. On a réparti les souris au hasard dans chaque série expérimentale. Une première expérimentation de routine a permis de

déterminer la mortalité par toxicité et de fixer ainsi les doses infra-létales.

Conformément à la convention en usage dans ce domaine, on considérait comme guéris les animaux qui survivaient plus de 45 jours après l'inoculation de cellules tumorales.

On a ainsi pu déterminer la mortalité par toxicité sous l'effet d'une injection unique de l'un des composés à différentes doses (doses infra-létales DL 50 et DL 100) et évaluer le taux de survie du groupe d'animaux auxquels on n'avait injecté qu'une dose infra-létale d'un composé selon l'invention.

On a calculé le taux de survie à partir d'une analyse statistique des résultats expérimentaux, par comparaison avec les résultats obtenus avec la série témoin, selon les méthodes bien connues de Mann-Whitey et de Wilcoxon ; le pourcentage de cellules leucémiques tuées par le traitement réalisé était calculé par la méthode décrite par H.E.Skipper et coll. dans les articles cités plus haut, en prenant pour base, suivant le cas, soit l'augmentation de la survie moyenne en l'absence de survivants, soit le pourcentage de survivants.

Pour l'évaluation de l'activité thérapeutique des composés selon l'invention, on a exprimé les doses desdits composés en les rapportant à la dose infra-létale 30 jours, que l'on a posée égale à 1. Cette dose infra-létale s'est en effet avérée plus facile à déterminer pour les composés de l'invention que la DL 10, dont elle est d'ailleurs très voisine et qui est habituellement prise comme référence pour l'évaluation de l'activité thérapeutique de composés à effet antitumoral (voir H.E. Skipper et coll., ouvrages cités).

En vue de la réalisation d'essais pharmacologiques, on a administré par voie intrapéritonéale à 20 souris femelles de souche DBA/2, âgées de 2 à 3 mois, un inoculum tumural de $10^5$ cellules de leucémie L 1210. Après 24 heures, on a fait à une moitié de ces souris une injection unique par voie intrapéritonéale d'une dose variable du composé respectif à tester (éventuellement mis au préalable sous la forme de l'un de ses sels hydrosolubles, si nécessaire), tandis qu'on injectait par la même voie à l'autre moitié des souris un même volume de solvant ; cette dernière moitié du groupe de souris servait donc de série témoin. On a réparti les souris au hasard dans chaque série expérimentale.

Les tableaux ci-après résument les résultats obtenus.

EFFETS IN VITRO ET IN VIVO DE QUELQUES COMPOSES SELON L'INVENTION.

| Composé | $DI_{50}$ * ($\mu M$) | $DL_0$ ** (mg/kg) | EFFICACITE ANTITUMORALE | |
|---|---|---|---|---|
| | | | Dose (fraction de $DL_0$) | ILS*** (I) |
| 10-hydroxy olivacine | 0,020 | 50 | 0,8 | 139 |
| 10-méthoxy olivacine | 1,091 | 150 | 0,66 | 147 |
| 4-$CH_3$ 10-OH olivacinium (sous forme acétate) | 0,562 | 100 | 1 | 155 |
| 4-β-diéthylaminoéthyl 10-OH olivacinium (acétate) | 0,236 | 125 | 0,8 | 182 (+4survivants sur 15) |

* Dose qui réduit de 50 % la croissance des cellules L1210, par rapport aux témoins, après 48 heures de culture.

** Dose maximale jamais mortelle

*** Increase in life Span (%) : augmentation de la durée de vie des sujets traités, T, par rapport aux témoins, C ($10^5$ cellules/souris par voie intrapéritonéale ; une injection unique i.p. 24 heures après la greffe de cellules leucémiques L1210), soit $\frac{T}{C} \times 100$

EFFET THERAPEUTIQUE DE DOSES VARIABLES (en injection unique, par voie intrapéritonéale) DE COMPOSES SELON L'INVENTION, POUR UN INOCULUM TUMORAL (i.p.) DE $10^5$ CELLULES DE LEUCEMIE L1210.

| Composé | Dose, fraction de la $DL_0$ | survie moyenne en jours des souris témoins | survie moyenne en jours des souris traitées | survivants | ILS*** (%) | % de cellules tumorales tuées. |
|---|---|---|---|---|---|---|
| 4-β-diéthyl-aminoéthyl | 0,5 | 9,7 ± 0,9 | 14,5 ± 2 | 0/10 | 150 | 99,98 |
| 10-hydroxy olivacinium (acétate) | 0,01 | 9,7 ± 0,9 | 11,6 ± 1,1 | 0/10 | 120 | 97 |
| | 1 | 9,3 ± 0,5 | 16,9 ± 3,7 | 4/15 | 182 | ≃ 99,999 |
| | 0,5 | 9,3 ± 0,5 | 22,3 ± 4,4 | 1/15 | 240 | ≃ 99,999 |
| $DL_0$ (i.p.) = 100 mg | 0,1 | 9,3 ± 0,5 | 15,1 ± 1,4 | 0/15 | 162 | 99,997 |
| | 0,05 | 9,3 ± 0,5 | 12,7 ± 0,4 | 1/15 | 136 | 99,8 |
| | 0,01 | 9,3 ± 0,5 | 11,9 ± 1,1 | 0/15 | 128 | 99 |
| | 0,005 | 9,3 ± 0,5 | 11,8 ± 0,8 | 0/15 | 127 | 99 |
| 10-hydroxy | 1 | 9,7 ± 0,9 | 14,4 ± 1,3 | 2/10 | 148 | 98,98 |
| olivacine (acétate) | 0,02 | 9,7 ± 0,9 | 10,5 ± 0,8 | 0/10 | non significatif | |
| | 1 | 9,2 | 11,8 | 0/15 | non significatif | |
| $DL_0$(i.p.) = 50 mg | 0,5 | 9,2 | 12,1 | 0/15 | non significatif | |
| | 0,1 | 9,2 | 10,9 | 0/15 | non significatif | |
| | 0,02 | 9,2 | 9,6 | 0/15 | non significatif | |

*** Increase in life Span (%) : augmentation de la durée de vie des sujets traités, T, par rapport aux témoins, C ($10^5$ cellules/souris par voie intrapéritonéale ;une injection unique i.p. 24 heures après la greffe de cellules leucémiques L1210), soit $\frac{T}{C} \times 100$.

D'une manière générale, les essais effectués ont montré que :

- l'hydroxylation en 10 des dérivés de l'olivacine se traduit par une forte augmentation de la cytotoxicité (d'un facteur de 86), nettement moins marquée cependant dans le cas de l'olivacine quaternarisée (facteur 4). En revanche, les toxicités générales ne varient pas (cas de l'hydroxylation en 10 de 4-$CH_3$ olivacinium) ou augmentent d'un facteur 5 (olivacine).

L'hydroxylation en 10 de l'olivacine ne modifie que peu les propriétés antitumorales à des doses voisines de la $DL_0$ ou égales à celle-ci, mais confère un index thérapeutique plus élevé au produit formé.

L'hydroxylation en 10 de 4-$CH_3$ olivacinium fait par contre apparaître des propriétés antitumorales que n'avait pas le composé de départ.

De ce qui précède, il ressort que l'augmentation très importante de la cytotoxicité, telle qu'elle a pu être déterminée in vitro, ne s'est pas traduite par un accroissement marqué de l'activité antitumorale chez l'animal, ni par une augmentation de la toxicité générale. Sans vouloir se lier à une quelconque théorie, on peut formuler, en guise d'explication, l'hypothèse que la pharmacocinétique et le métabolisme (glucuro-conjugaison) du dérivé hydroxylé en 10 sont tels, qu'une partie importante du composé à action médicamenteuse concerné est sequestrée ou éliminée après administration in vivo.

- la méthoxylation en 10 augmente la cytotoxicité. Par contre, la toxicité chez l'animal diminue après méthoxylation en 10, sauf après méthoxylation sur la position 10 de l'olivacine elle-même.

En ce qui concerne les propriétés antitumorales chez l'animal, la méthoxylation en 10 permet de les accroître, d'un facteur 1,3 dans le cas de l'olivacine et d'un facteur 1,7 dans le cas de la 4-méthyl olivacine.

- la quaternarisation de tous les dérivés d'olivacine (non substitués, 10-hydroxy ou 10-méthoxy) se traduit dans tous les cas par une diminution de la cytotoxicité (respectivement d'un facteur 1,4 pour le 4-méthyl olivacinium, d'un facteur 28,1 pour le 4-méthyl 10-hydroxy olivacinium et d'un facteur 1,3 pour le 4-méthyl 10-méthoxy olivacinium), et par une augmentation de la toxicité (d'un facteur 3,3 pour le 4-méthyl olivacinium et d'un facteur 1,5 pour le 4-méthyl 10-méthoxy olivacinium), sauf pour le 4-méthyl 10-hydroxy olivacinium avec lequel on a observé une diminution d'un facteur 2 de la toxicité.

La quaternarisation de l'olivacine elle-même s'est avérée entraîner une disparition de l'effet thérapeutique antitumoral. L'explication la plus vraisemblable qu'on puisse avancer est que l'efficacité thérapeutique passe par une métabolisation au niveau des microsomes, et qu'après cette quaternarisation une telle métabolisation de l'olivacine n'est plus possible.

En revanche, en ce qui concerne les dérivés 10-hydroxylés et 10-méthoxylés de l'olivacine, l'effet thérapeutique ne varie pas après quaternarisation.

Le cas de la 10-hydroxy olivacine quaternarisée est apparu comme remarquable, car on a pu établir (voir ci-dessus) qu'en quaternarisant la 10-hydroxy olivacine on la rend environ 30 fois moins cytotoxique, tandis que sa toxicité générale n'est abaissée que d'un facteur 2 et que l'efficacité

thérapeutique antitumorale n'est pas modifiée. Il est tout à fait remarquable et inattendu que cette variation considérable de cytotoxicité in vitro, après quaternarisation, ne se répercute ni sur la toxicité ni sur l'effet thérapeutique, qui restent à peu près les mêmes. Ces résultats suggèrent l'hypothèse que la 10-hydroxy olivacine quaternarisée aurait un métabolisme in vivo responsable de l'effet thérapeutique et de la toxicité générale qui n'apparaîtrait pas in vitro sur les cellules elles-mêmes.

L'invention a donc également pour objet une composition pharmaceutique anticancéreuse renfermant une quantité thérapeutiquement efficace d'un composé de formule I, sous forme d'une solution injectable ou sous forme d'une préparation solide administrable per os.

En thérapeutique humaine, le mode d'administration préféré de ces composés de formule I est l'injection par voie intraveineuse, à des doses et en respectant des dilutions du principe actif, éventuellement en mélange avec d'autres, que l'homme de l'art est à même de déterminer, à la suite d'un choix qu'il se fixe sur l'ampleur, la rapidité et le degré d'efficacité du traitement. En pratique, une dose d'environ 100 $mg/m^2$ de surface corporelle et par semaine peut être préconisée dans la plupart des cas.

Comme on l'a indiqué plus haut, on peut mettre en oeuvre lors de telles injections soit les sels quaternarisés, soit les bases libres elles-mêmes, soit les sels hydrosolubles que celles-ci forment par addition avec des acides pharmaceutiquement acceptables.

0042348

## REVENDICATIONS

1. Composés dérivés de l'olivacine, caractérisés en ce qu'ils répondent à la formule :

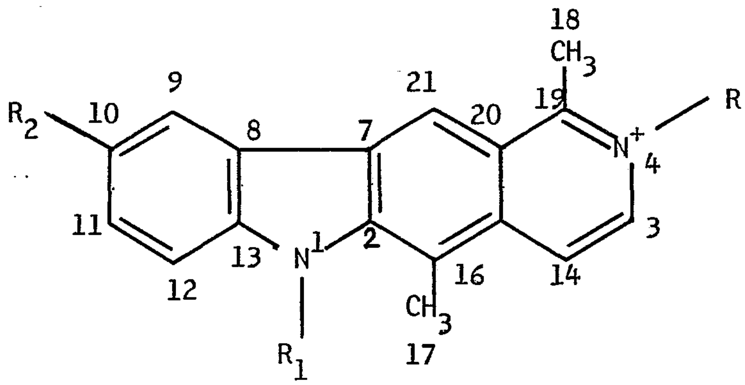

$X^-$ (I)

dans laquelle :

R est un atome d'hydrogène, ou un groupe alkyle, alkylamino ou aralkyle, substitué ou non, ou rien, et dans ce dernier cas l'atome d'azote en position 4 n'est pas quaternarisé ;

$R_1$ représente un atome d'hydrogène ou un groupe alkyle, alkylamino, ou aralkyle, substitué ou non ;

$R_2$ représente un atome d'hydrogène ou un groupe alcoxy ou hydroxy ;

à l'exception des cas où on a simultanément $R_1 = R_2 = H$ quel que soit R d'une part, et R = rien, $R_1 = H$, $R_2 = OH$ ou $OCH_3$ d'autre part ; et $X^-$ représente un anion d'acide minéral ou organique, ou rien et le composé correspondant est alors une base libre, ainsi que les sels d'addition des bases libres de formule I avec des acides pharmaceutiquement acceptables.

2. Composé selon la revendication 1, caractérisé en ce qu'il est choisi parmi les lactates et les acétates de :

1,4-diméthyl olivacinium,

10-hydroxy olivacinium,

4-méthyl 10-hydroxy olivacinium,

4-méthyl 10-méthoxy olivacinium

4-( β -diéthylaminoéthyl) 10-hydroxy olivacinium, et tout sel d'addition d'acide pharmaceutiquement acceptable des bases libres correspondantes.

3. Composition pharmaceutique anticancéreuse, caractérisée en ce qu'elle renferme une quantité thérapeutiquement efficace d'au moins un composé, éventuellement sous la forme d'un sel hydrosoluble, répondant à la formule

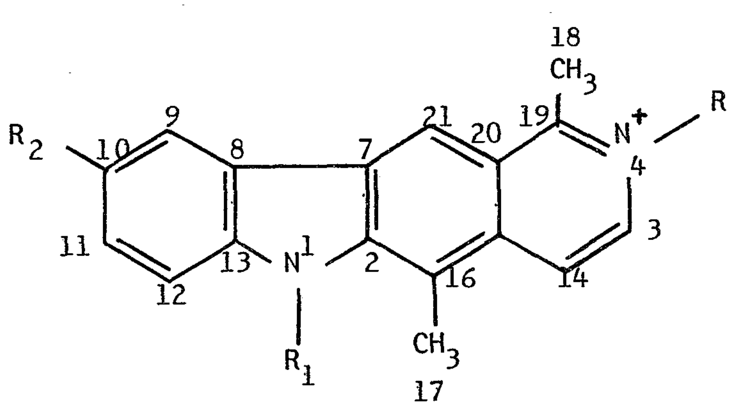

$X^-$ (I)

dans laquelle :

R est un atome d'hydrogène, ou un groupe alkyle, alkylamino ou aralkyle, substitué ou non, ou rien, et dans ce dernier cas l'atome d'azote en position 4 n'est pas quaternarisé ;

$R_1$ représente un atome d'hydrogène ou un groupe alkyle,alkylamino, ou aralkyle, substitué ou non ;

$R_2$ représente un atome d'hydrogène ou un groupe alcoxy ou hydroxy ;

à l'exception des cas où on a simultanément $R_1=R_2=H$ quel que soit R d'une part, et R= rien,$R_1$ = H, $R_2$ = $OCH_3$ d'autre part ; et $X^-$ représente un anion d'acide minéral ou organique, ou rien et le composé correspondant est alors une base libre, ainsi que les sels d'addition des bases libres de formule I

avec des acides pharmaceutiquement acceptables.

4. Composition pharmaceutique selon la revendication 3, caractérisée en ce qu'elle comprend au moins un composé choisi parmi les lactates et les acétates de :

1,4-diméthyl olivacinium,

10-hydroxy-olivacinium,

4-méthyl 10-hydroxy olivacinium,

4-méthyl-10-méthoxy olivacinium,

4-( β -diéthylaminoéthyl) 10-hydroxy olivacinium, ainsi que la 10-hydroxy olivacine base, et tout sel d'addition d'acide pharmaceutiquement acceptable des bases libres correspondantes.

5. Composition pharmaceutique selon la revendication 3, caractérisée en ce qu'elle est sous la forme d'une solution injectable.

0042348

Office européen des brevets

# RAPPORT DE RECHERCHE EUROPEENNE

Numéro de la demande
EP 81 40 0972

## DOCUMENTS CONSIDERES COMME PERTINENTS

| Catégorie | Citation du document avec indication, en cas de besoin, des parties pertinentes | Revendication concernée |
|---|---|---|
| X | JOURNAL OF CHROMATOGRAPHY, vol. 169, 1979 AMSTERDAM (NL) G. MUZARD et al.: "High-performance liquid chromatographic separation and trace determination of the antitumour derivatives of ellipticine and related quaternary ammonium compounds" pages 446-452 * Page 448, tableau II, composé 3 * | 1 |
| | CHEMICAL ABSTRACTS, vol. 82, 1975, réf.: 51486p, page 33 COLUMBUS, OHIO (US) & CIENC. CULT. (SAO PAULO) 1974, 26(5), 517-20 M. REGINA et al.: "Antineoplastic activity of olivacine" * En entier * | 1,4 |
| D | CHEMICAL ABSTRACTS, vol. 67, 1967 réf. 32869q, page 3127 COLUMBUS, OHIO (US) & CAN. J. CHEM. 45 (1), 89-92 (1967) R.H. BURNELL et al.: "Alkaloids of aspidosperma vargasii" * En entier * | 1 |
| A | FR - A - 2 308 365 (ANVAR) * Revendications * | |
| A | CH - A - 396 023 (Dr. A. WANDER AG) * Revendications * | |

CLASSEMENT DE LA DEMANDE (Int. Cl.³)
A 61 K 31/475
C 07 D 471/04//
(C 07 D 471/04
221/00, 209/00)

DOMAINES TECHNIQUES RECHERCHES (Int. Cl.³)
C 07 D 471/04

CATEGORIE DES DOCUMENTS CITES
- X: particulièrement pertinent
- A: arrière-plan technologique
- O: divulgation non-écrite
- P: document intercalaire
- T: théorie ou principe à la base de l'invention
- E: demande faisant interférence
- D: document cité dans la demande
- L: document cité pour d'autres raisons
- &: membre de la même famille, document correspondant

X Le présent rapport de recherche a été établi pour toutes les revendications

| Lieu de la recherche | Date d'achèvement de la recherche | Examinateur |
|---|---|---|
| La Haye | 16-09-1981 | VAN BIJLEN |